(19) Europäisches Patentamt
European Patent Office
Office européen des brevets

(11) **EP 4 151 140 A1**

(12) **EUROPEAN PATENT APPLICATION**

(43) Date of publication:
**22.03.2023 Bulletin 2023/12**

(21) Application number: **21196884.7**

(22) Date of filing: **15.09.2021**

(51) International Patent Classification (IPC):
**A61B 5/00** *(2006.01)*   **A61N 1/36** *(2006.01)*
**A61N 1/05** *(2006.01)*   **A61N 1/372** *(2006.01)*

(52) Cooperative Patent Classification (CPC):
**A61N 1/372; A61B 5/6885;** A61B 5/067; A61B 5/74;
A61B 34/30; A61B 46/10; A61B 2090/065;
A61B 2562/00; A61B 2562/0219; A61N 1/0541;
A61N 1/36039

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Medizinische Hochschule Hannover 30625 Hannover (DE)**

(72) Inventors:
• **RAU, Thomas Stephan 30853 Langenhagen (DE)**
• **BUDDE, Leon 30161 Hannover (DE)**
• **BÖTTCHER-REBMANN, Georg 30161 Hannover (DE)**
• **SCHELL, Viktor 30659 Hannover (DE)**

(74) Representative: **Gramm, Lins & Partner Patent- und Rechtsanwälte PartGmbB Freundallee 13a 30173 Hannover (DE)**

(54) **MEDICAL HANDLING SYSTEM**

(57)    The invention relates to a medical insertion system with an insertion device for inserting a medical device attached to the insertion device into a body region of a patient, wherein the insertion device has at least one force sensor for detecting a force transmitted from the medical device to the insertion device in a measuring direction, the insertion system having an evaluation unit for evaluating a force signal which is emitted by the force sensor and represents the force detected by the force sensor.

Fig. 1

**Description**

The invention relates to a medical handling system with a handling device for handling a medical device attached to the handling device into a body region of a patient, wherein the handling device has at least one force sensor for detecting a force transmitted from the medical device to the handling device in at least one measuring direction, the handling system having an evaluation unit for evaluating a force signal which is emitted by the force sensor and represents the force detected by the force sensor.

Medical handlings are performed on patients in various fields. The following is an example of the implantation of a cochlear implant, but the invention is also suitable for medical handlings in other areas.

The current standard surgical technique for inserting the electrode array of a cochlear implant is manual handling using forceps or tweezers. After exposure of the cochlea, the electrode array is manually positioned and advanced until it is either inserted sufficiently deep or strong, noticeable resistance causes the surgeon to abort the handling. Excessive forces during handling should be avoided because otherwise damage to the inner ear could occur.

There are already proposals to measure the insertion forces occurring during the insertion of a cochlear implant by means of a force sensor and to monitor them accordingly. Such an insertion system is known, for example, from WO 2009/124287 A1.

It is an object of the invention to provide a medical handling system with force sensing of a force transmitted to a handling device, in which improved and more precise force measurement is possible, in particular of very small forces.

This object is achieved by a medical handling system of the type mentioned above, characterized in that the evaluation unit is set up to determine a compensated force signal which is corrected for gravity and/or acceleration influences acting on the force sensor at least in the at least one measurement direction, which influence the force signal. In this way, the gravity and/or acceleration influences impacting the force measurement by the force sensor can be compensated.

Studies have shown that in the case of medical handlings in sensitive areas of the body, e.g. in the inner ear or on an eye, even relatively small forces can cause damage to the patient, in particular forces of a magnitude that the user (surgeon) can hardly feel or differentiate manually. It is therefore necessary, for example, for the handling of cochlear implants to detect forces in the range below 100 mN (millinewtons) with an accuracy of a few mN. For forces in this range, gravity effects and/or other acceleration effects acting on the force sensor can play a significant role. The proposed compensation of gravity and/or acceleration influences acting on the force sensor by means of the evaluation unit can significantly improve the measurement accuracy of the force signal. The invention therefore opens up useful application possibilities in the field of medical handlings in sensitive areas of patients' bodies. The medical handling system is suited for intraoperative use during a medical treatment of a patient.

The force sensor is set up to detect a force transmitted from the medical device to the handling device in at least one measuring direction. The at least one measuring direction can be implemented such that the handling force can be accurately measured, e.g. a force in the longitudinal direction of the medical device. In case of the insertion of a cochlear implant, the insertion force transmitted from the medical device to the handling device (insertion device) can be measured.

The invention is suitable for manual use of the handling device by the user as well as for robotic handling. The invention is suitable for static use in a fixture. For example, the handling device may be an insertion device for an electrode array of a cochlear implant. In this case, the medical device may be an electrode array of a cochlear implant. The handling device may also be configured for other areas of a patient's body, such as for intubation, for handling of a catheter or an endoscope, for performing optical coherence tomography (OCT) in an eye, or for handling of any kind of medical device in a surgical procedure. The medical device attached to the handling device may be, for example, a surgical device, a tube, a probe, or the like. The handling device has a mechanical connection interface for attaching the medical device to the handling device. The handling device may be equipped with medical devices of different kinds through the mechanical connection interface.

For determining the compensated force signal, the evaluation unit can be supplied with a measured and/or calculated gravity and/or acceleration signal, e.g. via an interface, which represents the gravity and/or acceleration acting on the force sensor at least in the measurement direction and influencing the force signal. The gravity signal may originate from a sensor arranged on or in the handling device. The gravity and/or acceleration signal may originate from another device, e.g., a stereooptical navigation system used to track the handling device. If the handling device is guided by means of a robot, the gravity and/or acceleration signal can be determined, for example, from spatial orientation information of the robot arm. In general, for determining the compensated force signal the evaluation unit can therefore be supplied with measured and/or calculated spatial orientation information, e.g. via an interface, which indicates the spatial orientation of the handling device in space. From this, the evaluation unit can calculate such a gravity signal to determine the compensated force signal. Depending on the way how the measured and/or calculated gravity and/or acceleration signal is generated, the evaluation unit may be supplied with additional data required for determining the compensated force signal, for example about the weight of the medical device and portions of the mechanical connection interface.

In an advantageous embodiment, the handling system is set up to detect a compensated force signal with a magnitude below 100 mN. A measuring accuracy of less than +/-10 mN can be achieved, or less than +/-5 mN, or less than +/-1 mN.

According to an advantageous embodiment of the invention, the handling device has at least one gravity sensor with which the gravity influences acting on the force sensor at least in the measuring direction can be determined in the form of a gravity signal, the evaluation unit being set up to determine the compensated force signal taking into account the gravity signal of the gravity sensor. This has the advantage that the gravity sensing is implemented directly in the handling device, whereby a high accuracy can be achieved. Such a gravity sensor can, for example, be designed as a commercially available acceleration sensor. This enables simple, precise and cost-effective determination of the gravity influences acting on the force sensor.

Therefore, it is possible that the handling device has at least one acceleration sensor with which the acceleration influences acting on the force sensor at least in the measuring direction can be determined in the form of an acceleration signal, the evaluation unit being set up to determine the compensated force signal taking into account the acceleration signal of the acceleration sensor

It is also possible to use a second force sensor for compensating the gravity and/or acceleration influences impacting the force measurement by the force sensor. In such case, the second force sensor can be connected to a weight simulation element which simulates the weight of the mechanical connection interface and portions of the medical device.

It is also possible that the handling device has more than one gravity sensor and/or acceleration sensor.

The determination of the compensated force signal taking into account the gravity and/or acceleration signal can, for example, be carried out mathematically as follows:

$$F_C = F_S - F_{ZP} + m_G\left(a - a_{ZP}\right)$$

Here Fc denotes the compensated force signal, Fs the force signal of the force sensor, $F_{ZP}$ a zero-point force signal, which represents an offset in the measurement. a is the gravity and/or acceleration signal of the gravity and/or acceleration sensor, $a_{ZP}$ is a zero-point signal of the gravity and/or acceleration sensor, i.e. an offset in the characteristic curve. The term $m_G$ represents the sum of the moving masses acting on the force sensor, which essentially means the total mass of the mechanical connection interface and portions of the medical device attached to the handling device.

According to an advantageous embodiment of the invention, the handling device has a multi-axis acceleration sensor by means of which the acceleration acting on the handling device in multiple spatial directions can be determined. This has the advantage that the spatial orientation of the handling device can be determined in several spatial axes at once, which in turn has the advantage that the current spatial orientation of the handling device can be displayed on a screen or other display device, for example, superimposed with an anatomical structure of the patient in the sense of an augmented reality display. For example, a multi-axis inertial measurement unit (IMU) can also be arranged on the handling device, in which a multi-axis rotation sensor (gyrometer) and/or a multi-axis magnetometer can also be arranged in addition to a multi-axis acceleration sensor. This makes it possible to determine the spatial position and spatial orientation of the handling device even more precisely.

According to an advantageous embodiment of the invention, the evaluation unit is set up to determine the compensated force signal by further considering gravity and/or acceleration signals and/or spatial orientation information occurring in a direction arranged at an angle to the measuring direction, e.g. with an angle greater than 5 degrees or an angle of about 90 degrees. This can further improve the accuracy of the determination of the compensated force signal. Depending on the design principle of the handling device and/or the force sensor, gravity and/or acceleration influences occurring at an angle to the measuring direction, for example, can falsify the signal of the force sensor. This influence can now also be compensated.

According to an advantageous embodiment of the invention, the handling system is arranged to display the compensated force signal on a display device, for example in real time. In this way, information about the forces transmitted to the patient in the area of a surgical intervention can be presented to the user simultaneously during the surgical intervention.

According to an advantageous embodiment of the invention, the handling system is arranged to output an acoustic, optical, and/or haptic feedback signal to the user depending on the compensated force signal. For example, there can be an acoustic, optical, and/or haptic feedback actuator on or in the handling device and/or any other part of the handling system. For example, one or more limit values can be stored in the handling system. If the compensated force signal reaches or exceeds one of these limit values, the feedback signal can be output to the user by activating such feedback actuator. If several limit values are available, different feedback signals can be output, for example, in a staggered manner for different force values, e.g. with increasing intensity for increasing forces.

It is possible, for example, to output a warning tone when a predefined limit value of the compensated force signal is exceeded, for example in the form of an acoustic reproduction of the compensated force signal, e.g. by increasing pitch, frequency modulation or the like. Also conceivable is a visual representation of the compensated force signal, e.g. by light sources, such as light emitting diodes, built into the handling device or another part of the handling system, which, for example, represent the intensity of the compensated force signal by changing colors. Haptic feedback is also conceivable, e.g. in the form of housing vibrations of the handling device or through a separate vibration device, e.g. on the user's arm. Likewise, visualization of the compensated force signal in the surgical microscope and/or visualization in the user's field of view is advantageously possible, e.g. as an augmented reality representation.

According to an advantageous embodiment of the invention, the handling system is arranged for storing the compensated force signal over time and/or for storing multi-axis spatial position and/or spatial orientation information of the handling device over time. This has the advantage that the compensated force signal and/or the multi-axis spatial position and/or spatial orientation information can be retrieved and checked for later verification of the medical handling process, for training purposes, and/or patient data documentation. For example, the stored data can be played back in the form of a video.

According to an advantageous embodiment of the invention, the handling device is an insertion device for inserting the medical device attached to the insertion device into a body region of a patient. In such case, the medical handling system can be a medical insertion system.

According to an advantageous embodiment of the invention, the insertion device has a mechanical connection interface for attaching the medical device in the form of an electrode array of a cochlear implant to the insertion device. This has the advantage that the insertion device can be used as an insertion tool for the electrode array of a cochlear implant.

For example, the invention is suitable for insertion force measurement in routine care of patients with cochlear implants. The invention is suitable for research into the relationship between measured forces during cochlear implant insertion and the patient's residual hearing retention. In addition, critical insertion forces and thresholds can be identified so that the cochlear implant insertion procedure can be improved in general.

The invention enables further advantages:

-    Reliable and meaningful handling force measurement by compensation of motion artifacts by means of sensor data fusion with at least one gravity and force sensing system.

    -    Can be used intraoperatively
    -    Research tool to develop a better understanding of the relationship between handling forces and residual hearing retention.

-    This enables realistic force feedback for users during handling for the

-    Control/monitoring of the handling process
-    early detection of mishandlings, damaging events
-    Adaptation of the handling process by reacting to the information provided, e.g. pause during insertion, slowing down of the movement, rotation of the electrode array, change of the handling angle.
-    Increase patient safety

-    Enabling correlation between orientation change and handling force

    -    Investigation of correlations between orientation change and handling force
    -    If correlation is identified: Additional feedback about unfavorable / too strong movements to the user

In an independent aspect, the present invention relates to a measuring device for measuring a mechanical physical quantity using a non-sterile sensor. The measuring device has a sterile barrier separating a non-sterile region from a sterile region. The non-sterile sensor is located in the non-sterile area of the measuring device. The measuring device has a transmission element for mechanically transmitting the mechanical physical quantity gathered in the sterile area to the non-sterile sensor. Thereby, the transmission element projects from the non-sterile sensor through an opening in the sterile barrier into the sterile area. The invention has the advantage that in a sterile environment, for example in a surgical or other medical environment, the measurement of mechanical physical quantities, for example of forces or force effects, can be carried out in a manner that is harmless to the patient. In this regard, the measuring device or at least parts of the measuring device, such as the sensor, need not themselves be sterilized, but may be sterilely enclosed by the sterile barrier, for example encapsulated therein. In this way, a general sterile concept is provided which can be used for sensory detection of mechanical quantities in sterile environments with little effort. The measuring device can be a medical handling system of the aforementioned kind. The transmission element can be a mechanical connection interface of the aforementioned kind.

The measuring device can be used, for example, to measure forces exerted by a surgical instrument on a patient's tissue. It is also possible to measure forces that occur when an implant is inserted inside the body as a result of contact with the surrounding tissue.

The measurement of the mechanical physical quantity, for example the force measurement, can advantageously be carried out through the opening in the sterile barrier. The mechanical physical quantity, for example the force, can thus be transmitted to the sensor unhindered and thus unmodified by other elements. In order that the opening in the sterile barrier does not lead to a violation of the sterility requirements in the sterile area, the sterile barrier formed in this way can be further enhanced by additional features, as explained hereinafter.

For example, the sterile barrier may comprise a flexible element, such as a sterile drape (e.g. foil, surgical drape), which envelops and shields at least the non-sterile parts of the measuring device from the sterile environment.

The sterile barrier may be specially designed, particularly in the region of the opening, to minimize the risk of contamination of the sterile region, for example by creating an angled path, for example in the sense of a labyrinth, through the opening in the sterile barrier. For example, an angled path in a gap between a sterile cover member, which covers the transmission element in the region of the opening, and the transmission element is advantageous.

The risk of contamination to be avoided includes

I.) the escape of non-sterile particles/droplets from the non-sterile interior of the sterile barrier through the opening into the sterile area and thus may cause e.g. contamination of the sterile area, as well as

II.) the entry of droplets of blood, secretions, rinsing fluid or other media from the sterile area into the interior of the sterile barrier, which can contaminate the measuring device there and/or come into contact with non-sterile surfaces there and flow back into the sterile area.

The measurement of the mechanical physical quantity requires a mechanically fixed, and advantageously also detachable, connection between the detection location of the mechanical physical quantity (e.g. instrument tip, implant) and the measuring device.

In the case of a (sterile) instrument, this can be connected directly to the (non-sterile) measuring device, e.g. by screwing it on, clamping it, pressing it in or similar, and thus itself acts as a transmission element for the force measurement. In the case of a (sterile) implant, this can be held by a rigid implant holder, which is connected to the measuring device and thus serves as a transmission element for the acquisition of the mechanical measured variable.

The sterile encapsulation of the measuring device by the sterile barrier, e.g. in the form of a rigid, single or multi-part housing, a sterile drape, foil or the like, includes an opening to allow direct connection to the sensing location of the mechanical physical quantity. The invention is thus characterized by (at least) one opening in the sterile barrier.

The transmission element can be guided through the opening without contact to surrounding elements, as otherwise frictional forces between the sterile barrier and instrument or between the sterile barrier and the transmission element would superimpose and falsify the measurement. The opening enables the transfer of a movement through the sterile barrier to the non-sterile sensor, which is necessary for the measurement. The movement may be very small (e.g. a few $\mu$m), as in the case of a force sensor based on the principle of a deformation body and strain gauge. However, the movement can also be large, at least along the longitudinal axis of the transmission element, if the force measurement is combined with, for example, a linear positioning movement.

In another aspect, the invention relates to an advantageous assembly sequence of the components of the measuring device:

  ∘ first the sterile sterile barrier is attached to the measuring device
  ∘ this results in a sterile outer surface and a space inside the sterile barrier which encloses non-sterile surfaces, such as outer surfaces of the measuring device as well as inner surfaces of the sterile barrier, in particular the non-sterile sensor
  ∘ the sterile transmission element is passed through the opening in the sterile barrier, whereby the (non-sterile) inner surfaces within the sterile barrier that are still accessible through the opening are now in turn covered in a sterile manner
  ∘ the geometric design of the transmission element, opening and sterile barrier already ensures at this stage that contact of non-sterile inner surfaces by the user is no longer possible

If the sterile encapsulation is not realized by a rigid housing alone, but by or with a flexible drape or foil, the gap geometry in the area of the opening must be realized by constructive design of the measuring device. This is possible, for example, by means of a projecting sleeve, opening socket or the like surrounding the mounting point for the transmission element, which is passed from the inside to the opening in the flexible sterile barrier or through the opening in the flexible sterile barrier and to which the foil or the drape can be temporarily fixed.

In the event that components of the measuring device, such as the above-mentioned projecting sleeve/opening socket are passed through, the non-sterile surfaces of the measuring device which are not yet sterilely covered by the sterile barrier must be covered by a further sterile part, e.g. in the form of a union nut, which thus forms part of the sterile barrier.

To minimize the risk of contamination, the gap in the remaining opening between the sterile barrier and the transmission element can be reduced by one or more of the following features:

  o constructive minimization of the gap width to the smallest possible dimension
  o longest possible gap length to create a high aspect ratio
  o and in particular by increasing the gap length by "twisting" the gap path

Such twisted or angled gap path makes it possible that there is no direct straight line connection from the sterile outer area to the first non-sterile inner surface, so that a direct entry and impact of droplets/splashes is prevented, as such droplet flight cannot occur "around the corner". By changing the direction of the gap several times up to a meandering design, the protective effect of the gap guide can be maximized and the risk of contamination can be reduced towards zero.

In this regard, a portion of the gap formed entirely by sterile surfaces may reduce the contamination risk II described above. A part of the gap that is also formed by non-sterile surfaces can reduce the contamination risk I described above.

The sterile barrier may be supplemented by one or more of the following features:

o Splash guard in the form of a plate or collar-shaped component which can be mounted on the transmission element and covers the remaining gap between the transmission element and the sterile barrier, thus additionally preventing droplets from flying,
o Positive or negative pressure inside the sterile barrier to create a defined flow direction in the "gap labyrinth"; the flow direction can be inward or outward, depending on whether contamination risk I or contamination risk II is to be reduced.

The invention is explained in more detail below with reference to examples of embodiments using drawings.

Figure 1    shows a schematic diagram of an insertion system,
Figure 2    shows a sectional side view of a part of an insertion device.

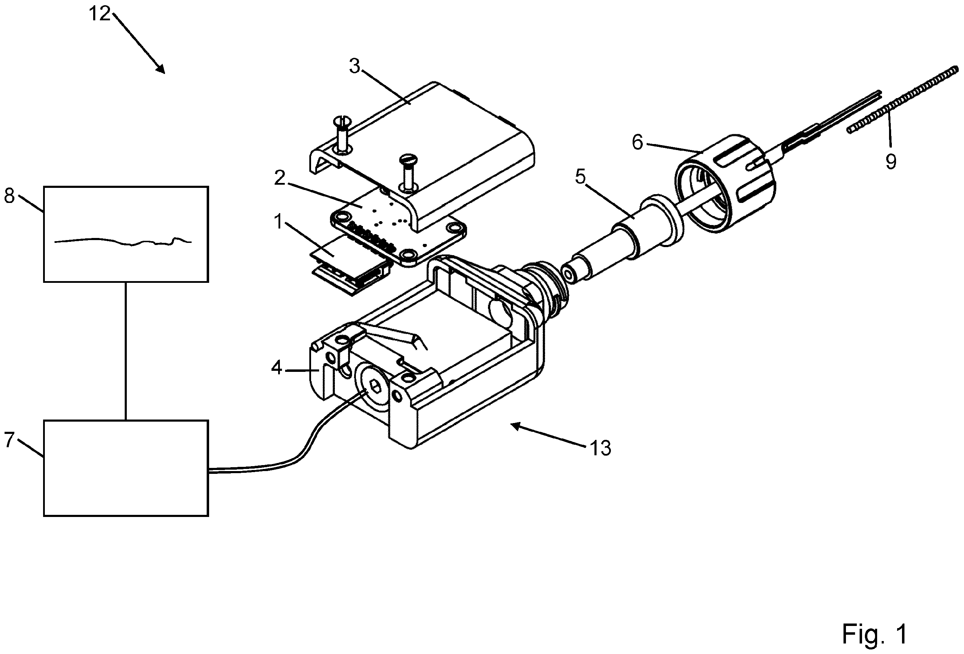

Figure 1 shows a medical insertion system 12 which has an insertion device 13, an evaluation unit 7, and a display device 8. The insertion device 13 can be designed, for example, as an insertion device to be held and guided manually. Alternatively, the insertion device 13 can also be guided by a robot.

The insertion device 13 has a multi-part housing with several parts, e.g. parts 3 and 4. The housing contains a force sensor 1 and a gravity sensor 2, e.g. in the form of a multi-axis multifunctional sensor unit in the sense of an inertial measurement unit (IMU). The force sensor 1 is mechanically coupled to a mechanical connection interface 5. A medical device 9 to be inserted into the patient by means of the insertion device 13 can be attached to the mechanical connection interface 5, for example, in the case of a cochlear implant, its electrode carrier. To achieve sterile conditions, part of the insertion device 13 and in particular the mechanical connection interface 5 can be covered by a closure cap 6.

The electrical or electronic components of the insertion device 13 are coupled to an evaluation unit 7 via electrical lines or wirelessly. The evaluation unit 7 is set up to evaluate the force signal emitted by the force sensor 1, the gravity signal emitted by the gravity sensor 2 and to determine a compensated force signal in which the gravity influences acting on the force sensor 1 are compensated by means of the gravity signal of the gravity sensor 2. The evaluation unit 7 is connected to a display device 8, for example a computer screen. The evaluation unit 7 is set up to display on the display device 8, for example, the compensated force signal in real time. The evaluation unit 7 can also output one or more feedback signals on the display device 8 or on other output components not shown here as a function of the compensated force signal, e.g. warning signals when certain predefined force values are reached.

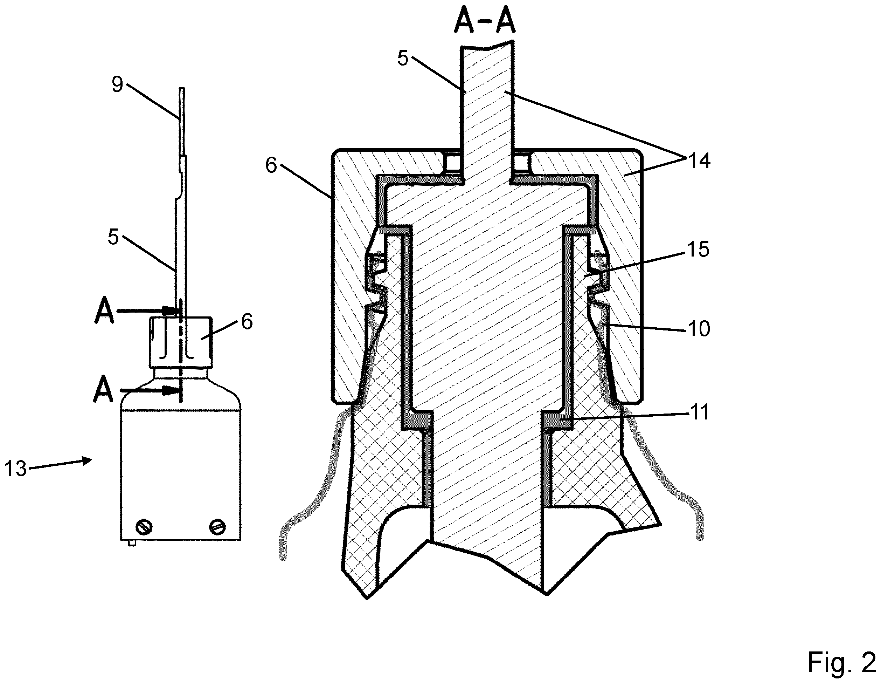

Figure 2 shows in the left hand area the insertion device 13 in a top view. In the right hand area of figure 2 the insertion device 13 facing the patient is shown in a sectional view along section line A - A. To enable the insertion device 13 to be used in accordance with regulations in the sterile operating room, some additional elements are added for separating sterile parts 14 from unsterile parts 15.

For example, some part of the insertion device 13 can be enclosed in a sterile cover (drape) 10. For example, a drape for use on endoscopes may be used. In the area of the mechanical connection interface 5, the sterile cover 10 is provided with a slight opening. To ensure a sterile barrier, the sterile closure cap 6 is screwed on in this area. The closure cap 6 thereby clamps the sterile cover 10 tightly and in this way prevents intraoperative slippage.

In addition, a labyrinth seal 11 may be located between the mechanical connection interface 5 and the housing 3, 4. Such a labyrinth seal 11 minimizes the risk of particles from falling out of the interior of the housing 3, 4 into the sterile area 14.

Since the insertion device 13 requires a through-opening from the sterile area where the medical device 9 is located to the force sensor within the housing 3, 4, it is required to provide a sterile concept which ensures that the sterile area of the medical device 9 is not contaminated through the opening from the area where the force sensor 1 is located.

The force sensor 1 has to be mechanically connected to the medical device 9, but shall not get in contact with other sterile elements. Therefore the non-sterile force sensor 1 needs to be separated from the sterile environment. This is done by the sterile cover 10 and the labyrinth seal 11. The insertion device 13 can be mounted in the following way.

1) Locating the insertion device 13 within the sterile cover 10;
2) Attaching the mechanical connection interface 5 on the insertion device 13;
3) Mounting the closure cap 6 on the insertion device 13;

4) Mounting the medical device 9 on the mechanical connection interface 5.

**Claims**

1. Medical handling system (12) with a handling device (13) for handling a medical device (9) attached to the handling device (13) within or relative to a body region of a patient, wherein the handling device (13) has at least one force sensor (1) for detecting a force transmitted from the medical device (9) to the handling device (13) in at least one measuring direction, the medical handling system (12) having an evaluation unit (7) for evaluating a force signal which is emitted by the force sensor (1) and represents the force detected by the force sensor (1), **characterized in that** the evaluation unit (7) is set up to determine a compensated force signal which is corrected for gravity and/or acceleration influences acting on the force sensor (1) at least in the at least one measurement direction, which influence the force signal.

2. Medical handling system according to claim 1, **characterized in that** the handling device (13) has at least one gravity sensor (2) with which the gravity influences acting on the force sensor (1) at least in the measuring direction can be determined in the form of a gravity signal, the evaluation unit (7) being set up to determine the compensated force signal taking into account the gravity signal of the gravity sensor (2).

3. Medical handling system according to claim 1 or 2, **characterized in that** the handling device (13) has at least one acceleration sensor (2) with which the acceleration influences acting on the force sensor (1) at least in the measuring direction can be determined in the form of a acceleration signal, the evaluation unit (7) being set up to determine the compensated force signal taking into account the acceleration signal of the acceleration sensor (2).

4. Medical handling system according to claim 3, **characterized in that** the handling device (13) has a multi-axis acceleration sensor (2) by means of which the acceleration acting on the handling device (13) in multiple spatial directions can be determined.

5. Medical handling system according to any one of the preceding claims, **characterized in that** the medical handling system (12) is arranged to display the compensated force signal on a display device (8).

6. Medical handling system according to any one of the preceding claims, **characterized in that** the medical handling system (12) is arranged to output an acoustic, optical and/or haptic feedback signal to the user depending on the compensated force signal.

7. Medical handling system according to any one of the preceding claims, **characterized in that** the handling system (12) is arranged for storing the compensated force signal over time and/or for storing multi-axis position and/or orientation information of the handling device in space over time.

8. Medical handling system according to any one of the preceding claims, **characterized in that** the handling device (13) is an insertion device for inserting the medical device (9) attached to the insertion device into a body region of a patient.

9. Medical handling system according to claim 8, **characterized in that** the insertion device has a mechanical connection interface (5) for attaching the medical device (9) in the form of an electrode array of a cochlear implant to the insertion device.

10. Medical handling system according to any one of the preceding claims, **characterized in that** the medical handling system (12) is set up to determine compensated force signals with a magnitude below 100 mN and/or with a measurement accuracy of below +/-10 mN .

Fig. 1

A-A

Fig. 2

EP 4 151 140 A1

**EUROPEAN SEARCH REPORT**

Europäisches
Patentamt
European
Patent Office
Office européen
des brevets

Application Number

EP 21 19 6884

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/225787 A1 (SIMAAN NABIL [US]; MANOLIDIS SPIROS [US]; UNIV COLUMBIA [US]) 27 September 2007 (2007-09-27) | 1,3-10 | INV.<br>A61B5/00<br>A61N1/36 |
| A | * the whole document * | 2 | |
| X | US 2010/094312 A1 (RUIZ MORALES EMILIO [IT] ET AL) 15 April 2010 (2010-04-15) | 1-4,6-8 | ADD.<br>A61N1/05<br>A61N1/372 |
| A | * the whole document * | 5,9,10 | |
| A | KOBLER JAN-PHILIPP ET AL: "An automated insertion tool for cochlear implants with integrated force sensing capability", INTERNATIONAL JOURNAL OF COMPUTER ASSISTED RADIOLOGY AND SURGERY, vol. 9, no. 3, 1 May 2014 (2014-05-01), pages 481-494, XP055896380, DE ISSN: 1861-6410, DOI: 10.1007/s11548-013-0936-1 Retrieved from the Internet: URL:https://link.springer.com/content/pdf/10.1007/s11548-013-0936-1.pdf> * abstract * * page 482, right-hand column, paragraph 3 - page 483, right-hand column, paragraph 2 * * page 484, right-hand column, last paragraph - page 485, left-hand column, paragraph 1 * * page 489, left-hand column, paragraph 1 - page 490, right-hand column, paragraph 2 * | 1-10 | |
| A | US 2013/138117 A1 (ABBOTT JACOB J [US] ET AL) 30 May 2013 (2013-05-30) * paragraph [0044]; figure 1 * | 1-10 | TECHNICAL FIELDS SEARCHED (IPC)<br>A61N<br>A61B |

-/--

The present search report has been drawn up for all claims

| Place of search | Date of completion of the search | Examiner |
|---|---|---|
| Munich | 2 March 2022 | Lorenz, Larissa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 1 of 2

Europäisches
Patentamt

European
Patent Office

Office européen
des brevets

# EUROPEAN SEARCH REPORT

**Application Number**

EP 21 19 6884

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,P | SCHELL VIKTOR ET AL: "A software for online monitoring of orientation-compensated forces during CI insertion", CURRENT DIRECTIONS IN BIOMEDICAL ENGINEERING, vol. 7, no. 2, 1 October 2021 (2021-10-01), pages 97-100, XP055896347, DOI: 10.1515/cdbme-2021-2025 * page 98, left-hand column, paragraph 1 * * page 98, left-hand column, paragraph 3 – page 98, right-hand column, paragraph 5 * ----- | 1-10 | |

| | | | TECHNICAL FIELDS SEARCHED (IPC) |
|---|---|---|---|
| | | | |

The present search report has been drawn up for all claims

| Place of search | Date of completion of the search | Examiner |
|---|---|---|
| Munich | 2 March 2022 | Lorenz, Larissa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

..................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 19 6884

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-2022

| Patent document cited in search report | | | Publication date | Patent family member(s) | | | Publication date |
|---|---|---|---|---|---|---|---|
| US | 2007225787 | A1 | 27-09-2007 | US | 2007225787 | A1 | 27-09-2007 |
| | | | | US | 2012109274 | A1 | 03-05-2012 |
| US | 2010094312 | A1 | 15-04-2010 | BR | PI0717408 | A2 | 21-01-2014 |
| | | | | BR | 122012021629 | A2 | 14-07-2015 |
| | | | | CA | 2664997 | A1 | 02-05-2008 |
| | | | | CA | 2870343 | A1 | 02-05-2008 |
| | | | | CN | 101528151 | A | 09-09-2009 |
| | | | | CN | 102697559 | A | 03-10-2012 |
| | | | | CY | 1116282 | T1 | 08-02-2017 |
| | | | | CY | 1116722 | T1 | 15-03-2017 |
| | | | | DK | 2083737 | T3 | 31-08-2015 |
| | | | | DK | 2491884 | T3 | 11-05-2015 |
| | | | | EP | 1915963 | A1 | 30-04-2008 |
| | | | | EP | 2083737 | A1 | 05-08-2009 |
| | | | | EP | 2491884 | A1 | 29-08-2012 |
| | | | | ES | 2535843 | T3 | 18-05-2015 |
| | | | | ES | 2546377 | T3 | 23-09-2015 |
| | | | | JP | 5044659 | B2 | 10-10-2012 |
| | | | | JP | 5540043 | B2 | 02-07-2014 |
| | | | | JP | 2010507792 | A | 11-03-2010 |
| | | | | JP | 2012254303 | A | 27-12-2012 |
| | | | | KR | 20090094234 | A | 04-09-2009 |
| | | | | KR | 20120099752 | A | 11-09-2012 |
| | | | | MX | 338904 | B | 03-05-2016 |
| | | | | PL | 2083737 | T3 | 29-02-2016 |
| | | | | PL | 2491884 | T3 | 31-07-2015 |
| | | | | PT | 2083737 | E | 28-09-2015 |
| | | | | PT | 2491884 | E | 11-06-2015 |
| | | | | RU | 2009119356 | A | 27-11-2010 |
| | | | | RU | 2012122482 | A | 10-12-2013 |
| | | | | US | 2010094312 | A1 | 15-04-2010 |
| | | | | US | 2013012930 | A1 | 10-01-2013 |
| | | | | US | 2018194013 | A1 | 12-07-2018 |
| | | | | US | 2020156259 | A1 | 21-05-2020 |
| | | | | WO | 2008049898 | A1 | 02-05-2008 |
| US | 2013138117 | A1 | 30-05-2013 | US | 2013138117 | A1 | 30-05-2013 |
| | | | | WO | 2011103059 | A2 | 25-08-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009124287 A1